## Europäisches Patentamt

## European Patent Office

## Office européen des brevets

(19)

(11) Numéro de publication: **0 077 760**
**A2**

(12)
# DEMANDE DE BREVET EUROPEEN

(21) Numéro de dépôt: **82830229.9**

(22) Date de dépôt: **07.09.82**

(51) Int. Cl.³: **A 61 F 5/03**

(30) Priorité: **19.10.81 IT 1181681 U**

(43) Date de publication de la demande:
**27.04.83 Bulletin 83/17**

(84) Etats contractants désignés:
**AT BE CH DE FR GB LI LU NL SE**

(71) Demandeur: **Giontella, Massimo**
**Costa dei Magnoli 4**
**I-50125 Firenze(IT)**

(72) Inventeur: **Giontella, Massimo**
**Costa dei Magnoli 4**
**I-50125 Firenze(IT)**

(74) Mandataire: **Martini, Lazzaro et al,**
**Ufficio Brevetti Ing. Lazzaro Martini Via Brunelleschi, 1**
**I-50123 Firenze(IT)**

(54) Bande de contention dynamique, en particulier pour le traitement des lombarthroses vertébrales.

(57) Pour obtenir un effet de poussée centripète et vers le haut de la colonne vertébrale on utilize un coussinet(2)creux, en matière élastique et imperméable à l'air, gonflable avec de l'air, qui est fixé au côté antérieur d'un support(1) à la façon d'une plaque rigide; le dit support(1) est relié à une bande(3) à appliquer autour du tronc du patient et dont les extremités libres sont munies d'au moins un système de fermeture que l'on peut régler. Le support(1)et le coussinet(2)ont un profil triangulaire avec base et hauteur de 20 cm maximum et avec un sommet dans le bas.

Fig. 1

EP 0 077 760 A2

Bande de contention dynamique, en particulier
pour le traitement des lonbarthroses vertébrales

L'invention présente concerne une bande de
contention dynamique, en particulier pour le
traitement des lombarthroses vertébrales.

On sait que l'arthrose, aussi bien naturelle
que précoce, est due aux altérations destructives du cartilage recouvrant les têtes osseuses
articulées, associées aux modifications secondaires des articulations.

On sait aussi que dans les arthroses vertébrales et plus particulièrement dans la discopathie, le disque intervertébral perd son élasti
cité et sa compacité, se fêle ou s'amincit de
façon inégale et par conséquent le corp vertébral situé au-dessus tend à glisser vers le
côté le plus mince du disque; en même temps le
diamètre du trou intervertébral se resserre
avec compression du plexus endorachidien et par
conséquent il y a apparition de la douleur dans
la zone innervée du nerf comprimé.

La thérapie de l'arthrose vertébrale se sert actuellement d'ins-truments statiques qui, sans modifier l'état dégénératif du disque, ont pour but de suppléer l'insuffisance musculaire paravertébrale par l'immobilisation de la zone atteinte de la colonne vertébrale dans un corset orthopédique.

Les types de corset actuellement connus sont très nombreux, mais tous sont caractérisés par le fait qu'ils sont construits avec une partie rigide qui est fixe et ayant un rôle exclusivement statique, c'est-à-dire s'opposant à l'affaissement progressif des corps vertébraux. L'invention présente a pour but de remédier à cet inconvénient.

Nous avons atteint ce résultat conformément à l'invention en adoptant l'idée d'utiliser un coussinet creux, avec une tunique élastique, gonflable avec de l'air comprimé de façon à obtenir un effet de poussée centripète et vers le haut de la colonne vertébrale. Ce coussinet est fixé à un support rigide de dimensions réduites, lui même relié à une bande de toile qui en permet son application au patient.

Les avantages obtenus par l'invention présente consistent essentiellement en ceci que la bande est adaptée au traitement de chaque segment de la partie dorso-lombosacrée de la colonne vertebrale; qu'elle permet le soutien dynamique de la colonne vertébrale grâce à l'intensité de la force de poussée réglable au choix du patient; qu'elle est réalisée en differentes mesures afin d'être adaptée aux differentes tailles du patient;

- 3 -

0077760

qu'elle est aisément applicable, a une réalisation simple et un coût réduit.

L'invention est exposée ci-après plus en détail à l'aide de dessins représentant seulement un mode d'exécution.

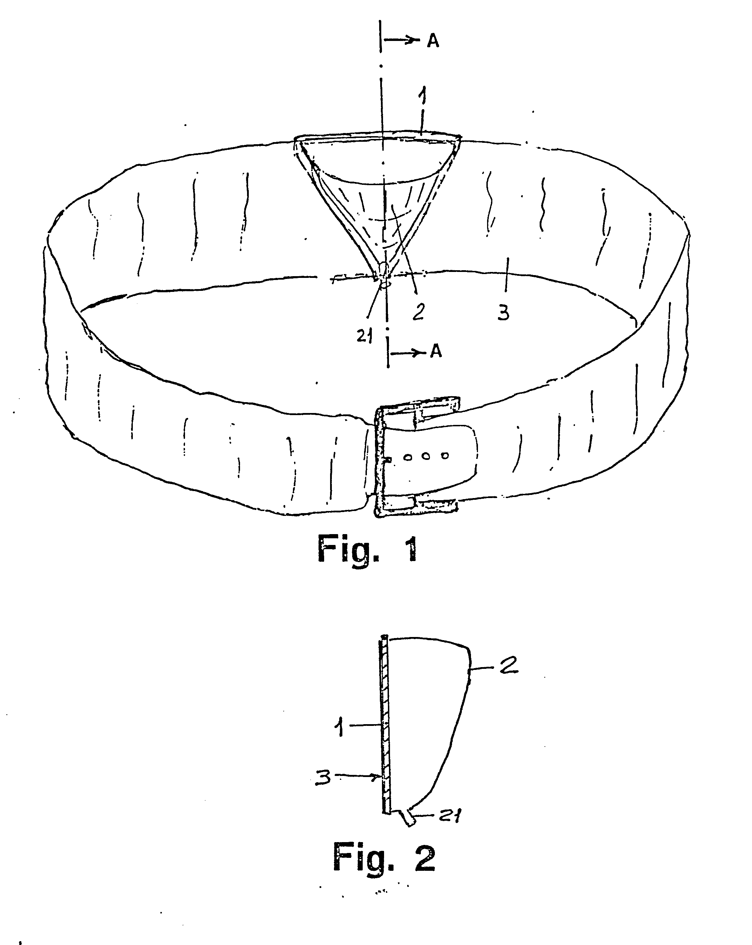

La Figure 1 représente une vue perspective d'ensemble d'une bande de contention dynamique conformément à l'invention en position d'utilisation.

La Figure 2 représente la coupe AA de la Figure 1. Réduite à sa structure essentielle et par référence aux dessins en annexe, une bande de contention dynamique, en particulier pour le traitement des lombarthroses selon l'invention est formée:

par un support (1) rigide, comme par ex.une plaque de matière plastique ou similaire de contour essentiellement triangulaire avec un sommet en bas;

par un coussinet (2) creux, sortant du côté antérieur du support(1) et dont le contour reprend principalement et coincide ou non avec celui du support (1), formé par une tunique élastique imperméable à l'air et donc gonflable avec de l'air sous pression par l'intermédiaire d'une canule(21) munie d'un robinet faisant partie avec le coussinet(2);

par une bande(3)de toile ou similaire, de hauteur égale ou supérieure au dit support(1) fixée au côté postérieur de celui-ci, de longueur suffisante pour envelopper le tronc du patient et avec les extrémités libres munies d'au moins un système de fermeture, tel une boucle ou similaire, pour boucler et serrer la bande(3)sur le tronc, en position diamètralement opposée par rapport au dit coussinet(2). Le coussinet(2) a la base et

- 4 -

**0077760**

l'hauteur de 20 cm.maximum.

Le fonctionnement est le suivant: après avoir bandé étroitement le tronc du patient avec la bande(3)et de façon que le coussinet(2)soit en contact avec la zone de la colonne à traiter et après avoir attaché les deux extrémités libres de la bande(3), on gonfle, avec des moyens connus, le coussinet(2)qui, ne pouvant pas s'etendre vers l'arrière de la bande(3)vu l'existence du support rigide(1), est obligé de s'étendre vers l'avant et c'est-à-dire contre la colonne vertébrale du patient dont les corps vertébraux, par rapport au niveau de la position du dit coussinet(2), reçoivent une poussée centripète et vers le haut entraînant ainsi une diminution de la compression des vertèbres sur les structures nerveuses liées aux parties osseuses. Avec un gonflement plus au moins important du coussinet (2)- qui peut être effectué directement par le patient à l'aide d'une simple pompe à piston reliée à la canule(21)- on peut obtenir une poussée utile plus ou moins grande sur les vertèbres.

REVENDICATIONS

1) Une bande de contention dynamique, en particulier pour le traitement des lombarthroses vertébrales caractérisée par le fait qu'elle est formée par un support(1)à la façon d'une plaque rigide, avec un côté antérieur d'où sort un coussinet(2)creux, en matière élastique etimperméable à l'air et avec un côté postérieur où est fixée une bande(3)en toile ou similaire à appliquer autour du tronc du patient et de façon que ledit coussinet(2)soit gonflé sous pression restant au contact de la zone de la colonne vertébrale à traiter.

2) Une bande de contention selon la revendication 1) caractérisée par le fait que le dit coussinet(2) a un profil de préférence triangulaire avec base et hauteur de 20 cm maximum et avec un sommet dans le bas en corrispondence duquel se trouve une canule(21) avec une valve d'étanchéité à commande manuelle pour remplir ou vider le coussinet(2).

3) Une bande de contention selon la revendication 1) caractérisée par le fait que le dit support(1) a un contour dépassant ou non celui du dit coussinet(2).

4) Une bande de contention selon la revendication 1) caractérisée par le fait que la hauteur de la dite bande(3)est au moins égale à celle du dit support(1)et dont les deux branches le dépassent d'une longueur égale ou non et dont les extrémités libres sont munies d'au moins un système de fermeture que l'on peut régler.

Fig. 1

Fig. 2